# EUROPEAN PATENT APPLICATION

(11) **EP 4 249 114 A1**
(43) Date of publication of application: **27.09.2023**
(21) Application number: 22163270.6
(22) Date of filing: 21.03.2022
(51) Int. Cl.: B01J 23/889, B01J 27/187, B01J 35/10, B01J 37/02, B01J 37/18, C07C 29/00, B01J 21/08

(54) **NICKEL-COPPER-MANGANESE HYDROGENATION CATALYST COMPRISING A SILICA SUPPORT**

(71) Applicant: BASF SE, 67056 Ludwigshafen am Rhein (DE)
(72) Inventor: HEIDEMANN, Thomas, 67056 Ludwigshafen (DE); STOCK, Christoph, 67056 Ludwigshafen (DE); PAPP, Rainer, 67056 Ludwigshafen (DE)
(74) Representative: Reitstötter Kinzebach

(57) **Abstract**

A hydrogenation catalyst has an active mass comprising nickel, copper and manganese deposited on a support comprising at least 90% by weight of silicon dioxide. The support has a BET surface area in the range from 190 to 370 m²/g. The catalyst has improved product selectivity in the catalytic hydrogenation of aldehydes to alcohols, and retains its high selectivity at the high temperatures.

## Description

The present invention relates to a hydrogenation catalyst, a method for preparing a hydrogenation catalyst and a method for producing an alcohol.

The catalytic hydrogenation of aldehydes or ketones to form saturated alcohols has been known for a long time. The reaction of an aldehyde with hydrogen generally is carried out in the presence of certain reduced metal compounds which act as hydrogenation catalysts. The commonly used commercial hydrogenation catalysts include copper chromite; cobalt compounds; nickel; nickel compounds which may contain small amounts of chromium or other promoters; mixtures of copper and nickel which may contain small amounts of chromium, manganese or other promoters; and a mixture of reduced copper oxide-zinc oxide.

DE 2 628 987 A discloses a supported catalyst for the hydrogenation of alkanals, wherein the active mass of the catalyst comprises from 40 to 80% by weight of nickel, from 10 to 50% by weight of copper and from 2 to 10% by weight of manganese. The hydrogenation process using this catalyst produced highly pure alkanols.

Further improvement of the process regarding the purity of the resulting alkanols was achieved by adding a base of an alkali or alkali earth metal, such as NaOH or KOH to the hydrogenation feed, see DE 10 024 542 A. DE 10 024 542A also discloses a variation of the catalyst of DE 2 628 987 A which is characterized by active mass comprising sodium oxide in addition to the metals nickel, copper and manganese (example 7).

EP 3 878 831 A1 provides a hydrogenation nickel-containing supported catalyst having an active mass comprising from 40 to 80% by weight of nickel, from 10 to 50% by weight of copper and from 2 to 10% by weight of manganese referred to the total mass of nickel, copper and manganese, which is characterized by a phosphorous content, calculated as atomic phosphor, of at least 0.2% by weight referred to the total mass of the nickel, copper and manganese of the catalyst.

All of these hydrogenation catalysts are associated with one or more disadvantages when used for commercially hydrogenating aldehydes to alcohols. Most of these catalysts exhibit a less than desired selectivity. Stated otherwise, when hydrogenating aldehydes using such catalysts, the quantity of by-products formed may be higher than desired. Such by-products reduce the desired selectivity of the aldehyde to alcohol conversion and generally must be removed from the hydrogenation product prior to subsequent use of the alcohol.

For example, in the catalytic hydrogenation of butyraldehyde to butanol over a nickel catalyst, small amounts of butyl ether and n-butyl butyrate form. Also, acetals from the aldehyde and the alcohol are frequently encountered. The ethers form azeotropes with the alcohol hydrogenation products and water frequently present in the product from the feed streams. Thus, a substantial amount of energy is required to separate by-product ethers from alcohols and significant losses of alcohol normally are encountered. For example, separation of butyl ether from butanol required for butanol to pass purity specifications, such as the specification for making acrylates, requires a series of costly distillation steps and because of the butyl ether-butanol azeotrope, some butanol is inevitably lost in the distillation. Such losses may render the use of an otherwise advantageous hydrogenation catalyst commercially unattractive.

While by-product esters may be easier to remove, the separation costs and associated losses are not inconsequential. Ester formation leads to a loss of alcohols via the ester stream purged from the bottom of the alcohol refining still. Furthermore, the amount of esters formed generally increases within increasing temperature in the catalytic hydrogenation reactor.

As is evident from the foregoing, a need exists in the art of catalytic hydrogenation of aldehydes to alcohols for a catalyst having improved product selectivity, particularly a catalyst which retains its high selectivity at the high temperatures needed to maximize reaction rates and energy efficiency.

The present invention relates to a hydrogenation catalyst having an active mass comprising nickel, copper and manganese deposited on a support comprising at least 90% by weight of silicon dioxide, wherein the support has a BET surface area in the range from 190 to 370 m²/g.

The invention also relates to a method for preparing a hydrogenation catalyst, comprising the steps of
(i) selecting a support comprising at least 90% by weight of silicon dioxide and having a BET surface area in the range from 190 to 370 m²/g;
(ii) impregnating the support with an aqueous solution of soluble salts of nickel, copper and manganese, optionally an aqueous solution of a soluble phosphate salt or phosphoric acid and optionally an aqueous solution of one or more soluble alkaline or alkaline earth salts,
(iii) drying the impregnated support,
(iv) optionally repeating steps (ii) and (iii), and
(v) calcining the dried impregnated support at a temperature in the range from about 500 to 600°C, to convert the metal salts to their oxides.

An important aspect of the new hydrogenation catalyst is its BET surface area. We believe that the higher BET surface area results in a better dispersion of the nickel, copper and manganese species. Thereby the hydrogenation reaction is accelerated more than undesired side reactions.

Preferably, the active mass comprises from 40 to 80% by weight of nickel, from 10 to 50% by weight of copper and from 2 to 10% by weight of manganese, based on the total mass of nickel, copper and manganese.

The active mass components nickel, copper and manganese of the hydrogenation catalyst may be present in form of chemical compounds, preferably as oxides, or completely or partially in metallic form. The latter is the active form of the hydrogenation catalyst.

In an embodiment the active mass has a phosphorous content of at least 0.2% by weight, based on the total mass of the nickel, copper and manganese. Usually, the phosphorous content, calculated as atomic phosphor, is in the range from 0.2 to 3.0% by weight, preferably in the range from 0.5 to 2.5% by weight, and more preferably in the range of 1.0 to 2.0% by weight, based on the total mass of the nickel, copper and manganese of the catalyst. The phosphorous content of the supported catalyst may be present in form of any phosphorous compound or in any form of elementary phosphor. Preferably the phosphorous content is present completely or partially in form of phosphor(V) compounds, such as phosphoric acid, salts thereof (e.g. sodium or potassium phosphate) or phosphor(V) oxide, or in form of elementary phosphor, or mixtures thereof. More preferably the phosphorous content is present completely or partially in form of phosphoric acid or phosphor(V) oxide or elementary phosphor, or mixtures thereof.

In a preferred embodiment of the invention, the active mass of the catalyst comprises at least one alkali or alkali earth oxide, more preferably at least one alkali oxide, most preferably sodium oxide. The active mass of the catalyst can comprise up to 5% by weight, preferably up to 3% by weight of such alkali or alkali earth oxide in total referred to the total mass of the nickel, copper and manganese. Preferably, the total content of such further alkali or alkali earth oxide is in the range from 0.1% to 3.0% by weight, more preferably in the range from 0.5 to 2.5% by weight, and most preferably in the range of 1.0 to 2.0% by weight, based on the total mass of the nickel, copper and manganese. In a particular embodiment, the active mass of the catalyst comprises sodium oxide in the range from 0.1% to 3.0% by weight, more preferably in the range from 0.5 to 2.5% by weight, and most preferably in the range of 1.0 to 2.0% by weight, based on the total mass of the nickel, copper and manganese.

The catalyst support comprises at least 90% by weight of silicon dioxide, preferably at least 98% by weight of silicon dioxide. The remainder of the support material may be another oxidic material, e.g. MgO, CaO, TiO₂, ZrO₂, Fe₂O₃ and/or an alkali metal oxide.

The catalyst support has a BET surface area in the range from 190 to 370 m²/g. Preference is given to supports which have a BET surface area in the range from 240 to 370 m²/g, preferably from 300 to 360 m²/g, preferably from 310 to 355 m²/g. The BET surface area as used herein is the BET surface area measured in accordance with DIN ISO 9277/DIN 66131.

Suitable support materials based on silicon dioxide are commercially available: Particular preference is given to Perlkat 97-0 (Siliperl AF 125) from BASF SE.

Depending on the way in which the hydrogenation is carried out, the support can have various forms. If the process is carried out as a suspension process, the support material will usually be used in the form of finely divided powder for producing the catalysts of the invention. The powder preferably has particle sizes in the range from 1 to 200 µm, in particular from 1 to 100 µm. When the catalyst is used in fixed beds, it is usual to employ shaped bodies made of the support material which are obtainable, for example, by extrusion, ram extrusion or tableting and can have, for example, the shape of spheres, pellets, cylinders, extrudates, rings or hollow cylinders, stars and the like. The dimensions of these shaped bodies are usually in the range from 1 mm to 25 mm. Catalyst extrudates having extrudate diameters of from 1.5 to 5 mm and extrudate lengths of from 2 to 25 mm are frequently used.

The silicon dioxide support material is particularly preferably used in the form of spherical shaped bodies for producing the catalyst. The spherical shaped bodies preferably have a diameter in the range from 1 to 6 mm, more preferably from 2 to 5.5 mm, in particular from 3 to 5 mm.

The shaped bodies, in particular the spherical shaped bodies, preferably have a (lateral) compressive strength of >60 newton (N), preferably >70 N, more preferably >80 N, more preferably >100 N, e.g. in the range from 90 to 150 N.

To determine the (lateral) compressive strength, the catalyst body was, for example, loaded on the cylindrical surface with increasing force between two parallel plates or, for example, the catalyst sphere was loaded with increasing force between two parallel plates until fracture occurred. The force recorded on fracture is the (lateral) compressive strength. The determination was carried out on a test instrument from Zwick, Ulm, having a fixed turntable and a freely movable, vertical punch which pressed the shaped body against the fixed turntable. The freely movable punch was connected to a load cell for recording the force. The instrument was controlled by means of a computer which recorded and evaluated the measured values. 25 shaped bodies which were in good condition (e.g. without cracks and, if appropriate, without broken edges) were taken from a well-mixed catalyst sample, their (lateral) compressive strength was determined and subsequently averaged.

The silicon dioxide support used for producing the catalyst particularly preferably has a water absorption (DIN 66134) in the range from 0.7 to 1 ml/g, preferably from 0.75 to 0.95 ml/g.

Typically, the supported catalyst contains, for example, from 3 to 50% by weight, preferably from 10 to 40% by weight, more preferably from 15 to 30% by weight (expressed as metal) of the active mass components nickel, copper and manganese.

The supported catalysts of the invention may be manufactured, for example, by impregnating the support with an aqueous solution of soluble salts of nickel, copper and manganese (e.g. nickel nitrate, copper nitrate and manganese nitrate), an aqueous solution of a soluble phosphate salt or phosphoric acid and optionally an aqueous solution of one or more soluble alkaline or alkaline earth salts, preferably sodium salts (e.g. sodium nitrate) containing the phosphorous and the metals in proportion to achieve the above percentages in the active mass, then drying the impregnated material. The impregnated material is then heated, e.g. in air at a temperature from about 500 to 600°C (calcination). This process can be repeated until the desired amount of active mass has been applied to the support.

After such calcination and before being used for the hydrogenation reaction, the nickel, copper and manganese oxides of the supported catalyst are then reduced to the active metal form, e.g. in the presence of hydrogen at a temperature in the range from 280 to 300°C. The reduction can be carried out in the hydrogenation reactor but in general it is more economical to use a separate apparatus for the reduction. Usually a stream of nitrogen and hydrogen is applied to the supported catalyst at a pressure of 1 to 10 bara, and the ratio of hydrogen is continuously increased up to 100% during this hydrogenation step. After cooling down to ambient temperature, the surface of the catalyst material can be passivated to allow better handling of the catalyst in the presence of air, e.g. by applying a mixture of nitrogen and air, starting with a very low air content (e.g. 0.1 vol-%) and being increased slowly up 100 vol-%, while the temperature of the catalyst should not exceed a temperature of 80°C, preferably of 50°C.

The invention further relates to a method for producing an alcohol by contacting a liquid hydrogenation feed, which comprises at least one aldehyde or ketone, with a hydrogen-containing gas in the presence of the hydrogenation catalyst according to the invention.

Preferably, the reaction temperature is in the range from 100 to 300°C. The pressure may be in the range from 1 to 700 bar.

The hydrogenation process according to the invention can be carried out either batchwise or continuously, for example with the aid of tubular reactors or reactor cascades. The catalyst bed generally rests on a suitable retention element in the reactor. The hydrogenation reactor can be operated either by the pool or trickle method. The hydrogenation process according to the invention is preferably carried out in a reactor cascade, in particular a cascade comprising two to five reactors.

The hydrogenation catalyst is generally loaded with from 0.01 to 2, preferably, with from 0.1 to 1 and in particular with from 0.2 to 0.5 L of aldehyde/L of supported catalyst per hour.

The addition of water to the hydrogenation feed is possible in the process according to the invention but is not necessary. If desired, 1 to 2 % by weight of water can be added. The hydrogenation feed generally contains traces of water, for example in the range from 1 ppm to 1% by weight, which have been introduced by the starting materials in the preceding synthesis steps or formed by condensation reactions. These traces of water have no substantial impact on the hydrogenation process according to the invention.

The liquid hydrogenation feed can consist of one or more undiluted aldehydes or ketones. However, in a particular embodiment the aldehydes or ketones are employed as a solution in an inert diluent. Examples of suitable inert diluents are hydrocarbons, ethers, such as diethyl ether, or alcohols. The diluents are particularly preferably alcohols, in particular the alcohol which is the hydrogenation product of the aldehyde to be hydrogenated. In a preferred embodiment, part of the hydrogenation product is recycled for this purpose and mixed with the aldehyde to be hydrogenated. If used, the inert diluent is preferably used in an amount of from 0.1 to 100 parts by weight, in particular from 1 to 50 parts by weight and particularly preferably from 5 to 20 parts by weight, based on 1 part by weight of aldehyde and ketone employed. If the hydrogenation is carried out adiabatically, i.e. with removal of the heat of reaction by the reaction product, the amount of inert diluent used is advantageously such that the temperature gradient over the bed of the granular catalyst does not exceed a temperature of 40°C. If, by contrast, the hydrogenation reactor is operated isothermally, the proportion of inert diluent in the hydrogenation feed can be selected virtually as desired.

In a preferred embodiment of the hydrogenation process, the hydrogenation feed comprises an amount of an alkali metal or alkali earth metal salt-like base with a basic anion. Preferably, the pKs value of the corresponding acid of the basic anion is higher than 2, preferably higher than 4, in particular higher than>8). Suitable basic anions are hydroxide, carbonate, hydrogencarbonate, phosphate, amide, hydride; alkoxides, in particular C₁-C₄-alkoxides, such as methoxide, ethoxide, n-propoxide and isopropoxide and butoxide; phenoxide, carboxylates, such as acetate or benzoate; carbanions, such as butyl, cyclopentadienyl or phenyl. In general, hydroxide or carbonate is preferred.

Advantageous salt-like bases are, in particular, alkali metal hydroxides and/or carbonates, such as lithium carbonate, potassium carbonate, sodium carbonate, lithium hydroxide, sodium hydroxide and potassium hydroxide. In general, sodium hydroxide and/or potassium hydroxide are preferred. However, sodium alkoxides and/or potassium alkoxides, such as the methoxide or ethoxide, or the alkoxide of the alcohol which is the hydrogenation product of the aldehyde present in the hydrogenation feed can also be employed with particular advantage.

The salt-like bases are generally added to the hydrogenation feed in an amount which corresponds, on neutralization equivalent basis, to the range from 0.1 to 2000 ppm by weight, preferably from 0.1 to 1000 ppm by weight, in particular from 0.1 to 100 ppm by weight, particular preferably from 0.5 to 50 ppm by weight and in particular from 1 to 20 ppm by weight, based on the aldehyde and ketone present in the hydrogenation feed, of potassium hydroxide. In the case of monovalent basic anions, a molar amount of salt-like base which corresponds to the stated amount of potassium hydroxide is used, and in the case of divalent basic anions, half the molar amount is used. It is also possible to add mixtures of different bases.

The resulting alcohols of the hydrogenation process can be further worked up by means of distillation.

The aldehydes and ketones to be hydrogenated according to the invention are not subject to any restrictions in principle. Preferably, aldehydes are employed for the hydrogenation according to the invention. The aldehydes which can be employed are preferably aliphatic C₂- to C₂₀-, in particular C₃- to C₁₅-aldehydes, which may be straight-chain or branched and may additionally contain C=C double bonds in the molecule. Suitable aldehydes which are of particular economic importance are, for example, acetaldehyde, propanal, n-butanal, isobutyraldehyde, crotonaldehyde, pentanal, 2-ethyl-4-methylpentenal, hexanal, 2-ethylhexanal, 2-ethylhexenal, 2-propylheptanal, 2-propylheptenal, octanal, nonanal, decanal, decenal, benzaldehyde, 2-phenylacetaldehyde, cinnamaldehyde, p-chlorobenzaldehyde, p-methoxybenzaldehyde and the hydroformylation products of trimeric and tetrameric propylene and dimeric and trimeric butene.

The hydrogen-containing gas preferably comprises more than 80 mol-% of hydrogen; in particular, it essentially consists of hydrogen. The hydrogen-containing gas can be passed over the fixed bed of the supported catalyst in co-current or counter-current to the hydrogenation feed. It is preferably passed in co-current. The amount of hydrogen-containing gas fed in is advantageously such that from 1.0 to 1.15 times the stoichiometrically necessary amount of hydrogen is available.

The invention is illustrated by the examples that follow.

### Examples

### Example 1

### Preparation of the catalysts

A reference catalyst (catalyst A) was prepared by mixing 1.22 kg of nickel nitrate solution (13.5% by weight of nickel), 0.35 kg of copper nitrate solution (15.5% by weight of copper), 0.11 kg of manganese nitrate solution (12.5% by weight of manganese), 59 g of sodium nitrate solution (4.1% by weight of sodium) and 15 g of phosphoric acid having a concentration of 75% (23.7% by weight of phosphor) and immersing 100 g of silica support material (SiO₂ strands having a diameter of 4 mm, a SiO₂ content of >99%, a BET surface area of 120 to 160 m²/g and a water absorption of 0.95 to 1.0 ml/g; bulk density 440-480g/l; type D11-10 by BASF SE) in this aqueous mixture for 30 min. After dripping off excessive solution, the impregnated material was dried over night at a temperature of 120°C and subsequently calcinated at a temperature of 600°C for a period of 2 h.

This process of immersing, drying and calcination was repeated with the catalyst material a second time. The resulting catalyst material contained 21.3% by weight of nickel, calculated as NiO, 7.3% by weight of copper, calculated as CuO, 2.0% by weight of manganese, calculated as Mn₃O₄, 0.35% by weight of sodium, calculated as Na₂O, and 1.2% by weight of phosphor, calculated as H₃PO₄, remainder SiO₂. This corresponds to an active mass with a weight ratio of 70 : 24 : 6 for the active mass components nickel: copper: manganese, and with 1.5% by weight of sodium oxide and 1.6% by weight of atomic phosphor, both based on the total of weight of nickel, copper and manganese.

An inventive catalyst (catalyst B) was prepared by the same procedure but using Perlkat 97-0 as support material. Perlkat 97-0 consists of SiO₂ spheres of 3 to 5 mm diameter, tempered at 950°C, a water absorption of 0.7-0.8 ml/g and a BET surface area of 240 to 300 m²/g; bulk density 500-600 g/l.

The higher bulk density of Siliperl AF 125 is largely compensated by the lower water absorption as compared to D11-10. This results in two catalysts with comparable amounts of the active components Ni/Cu/Mn/P/Na in a given reactor volume. Any differences in performance can therefore be reasonably related to the supports.

After cooling down to ambient temperature, the catalysts (catalyst A and B) were installed in reduction devices where the active mass was reduced by a mixture of nitrogen and hydrogen at a temperature of 250°C and at a pressure of 1 to 10 bara for 24 h. During this reduction step, the ratio of hydrogen within the stream of nitrogen and hydrogen was increased continuously from 1 to 100 vol-%. After cooling down to ambient temperature again, the surface of the catalyst material was passivated to allow better handling of the catalyst in the presence of air, by applying a mixture of nitrogen and air, starting with an air content of 0.1 vol-% and being increased slowly up 100 vol-%, in a way that the temperature of the catalyst does not exceed 35°C.

### Example 2

### Catalytic hydrogenation of n-butanal to n-butanol

A continuous flow hydrogenation apparatus was charged with 190 ml of activated catalyst A or B, respectively. The reaction conditions were 35 bar, 130°C or 150°C and the feed to recycle ratio was 1:12. n-butyric aldehyde from a hydroformylation was the hydrogenation feed.

To the feed 15 ppm KOH, dissolved in butanol were added. At a load of 0.42 kg/l*h and 130°C or 150°C, 35 bar, n-butyric aldehyde was hydrogenated to n-butanol.

**Results at 130 °C**

| | Catalyst A | Catalyst B |
|---|---|---|
| n-Butanol | 95,78 % | 96,8 % |
| Dibutylether | 0,18 % | 0,11 % |
| Butyl butyrate | 0,02 % | 0,02 % |
| Ethylhexandiol | 0,24 % | 0,22 % |
| Butylaldehyde dibutylacetal | 2,35 % | 1,57 % |

**Results at 150 °C**

| | Catalyst A | Catalyst B |
|---|---|---|
| n-Butanol | 98,1 % | 98,8 % |
| Dibutylether | 0,29 % | 0,12 % |
| Butyl butyrate | 0,02 % | 0,02 % |
| Ethylhexandiol | 0,47 % | 0,31 % |
| Butyraldehyde dibutylacetal | 0,79 % | 0,19 % |

## Claims

1. A hydrogenation catalyst having an active mass comprising nickel, copper and manganese deposited on a support comprising at least 90% by weight of silicon dioxide,
wherein the support has a BET surface area in the range from 190 to 370 m²/g.

2. The hydrogenation catalyst of Claim 1, wherein the active mass comprises from 40 to 80% by weight of nickel, from 10 to 50% by weight of copper and from 2 to 10% by weight of manganese, based on the total mass of nickel, copper and manganese.

3. The hydrogenation catalyst of Claim 2, wherein the active mass has a phosphorous content of at least 0.2% by weight, based on the total mass of the nickel, copper and manganese.

4. The hydrogenation catalyst of any one of the preceding Claims, wherein the active mass further comprises up to 5% by weight of alkali or alkali earth oxide, based on the total mass of the nickel, copper and manganese.

5. The hydrogenation catalyst of claim 4, wherein the alkali or alkali earth oxide is sodium oxide.

6. A method for preparing a hydrogenation catalyst, comprising the steps of
(i) selecting a support comprising at least 90% by weight of silicon dioxide and having a BET surface area in the range from 190 to 370 m²/g;
(ii) impregnating the support with an aqueous solution of soluble salts of nickel, copper and manganese, optionally an aqueous solution of a soluble phosphate salt or phosphoric acid and optionally an aqueous solution of one or more soluble alkaline or alkaline earth salts,
(iii) drying the impregnated support,
(iv) optionally repeating steps (ii) and (iii), and
(v) calcining the dried impregnated support at a temperature in the range from about 500 to 600°C, to convert the metal salts to their oxides.

7. The method of claim 6, which comprises the subsequent step of
(vi) reducing the nickel, copper and manganese oxides at least partially to the elemental metal form.

8. A method for producing an alcohol by contacting a liquid hydrogenation feed which comprises at least one aldehyde or ketone, with a hydrogen-containing gas in the presence of the hydrogenation catalyst of any of claims 1 to 5.

9. The method of claim 8, wherein the reaction temperature is in the range from 100 to 300°C.

10. The method of claim 6 or 7, wherein the pressure is in the range from 1 to 700 bar.

11. The method of any of claims 8 to 10, wherein hydrogenation feed comprises an aldehyde selected from acetaldehyde, propanal, n-butanal, isobutyraldehyde, crotonaldehyde, pentanal, 2-ethyl-4-methylpentenal, hexanal, 2-ethylhexanal, 2-ethylhexenal, 2-propylheptanal, 2-propylheptenal, octanal, nonanal, decanal, decenal, benzaldehyde, 2-phenylacetaldehyde, cinnamaldehyde, p-chlorobenzaldehyde, p-methoxybenzaldehyde and the hydroformylation products of trimeric and tetrameric propylene and dimeric and trimeric butene.

12. The method of any of claims 8 to 11, wherein the hydrogenation feed comprises water.
